Europäisches Patentamt

European Patent Office

Office européen des brevets

(19)

(11) Publication number: **0 125 858**
**A1**

(12) **EUROPEAN PATENT APPLICATION**

(21) Application number: **84303055.2**

(22) Date of filing: **08.05.84**

(51) Int. Cl.³: **A 61 K 31/635**
**//(A61K31/635, 31/155)**

(30) Priority: **06.05.83 GB 8312615**

(43) Date of publication of application:
**21.11.84 Bulletin 84/47**

(84) Designated Contracting States:
**CH DE FR GB IT LI NL SE**

(71) Applicant: **University of Strathclyde**
**McCance Building 16 Richmond Street**
**Glasgow, Scotland(GB)**

(72) Inventor: **Richards, Robert Michael Edward**
**Banehurst The Avenue**
**Bushey Watford, WD2 2LL(GB)**

(74) Representative: **Huskisson, Frank Mackie et al,**
**FITZPATRICKS 4 West Regent Street**
**Glasgow G2 1RS Scotland(GB)**

(54) Improved compositions for treating infected burn wounds.

(57) A pharmaceutical composition comprises in admixture with a pharmaceutically acceptable vehicle or carrier, a therapeutically effective amount of a silver compound suitable for treating burns, preferably silver sulphadiazine, and a synergistic amount of dibromopropamidine or pharmaceutically acceptable salts thereof, preferably the isethionate or pamoate.

EP 0 125 858 A1

- 1 -

"Improved Compositions for Treating Infected Burn Wounds"

This invention relates to pharmaceutical compositions containing silver compounds. More particularly it relates to silver compounds suitable for use in the treatment of burn wound infection.

Silver sulphadiazine is the most commonly used compound in pharmaceutical compositions for treating patients having burn wounds infected by Pseudomonas aeruginosa, Enterobacter cloacae and Staphylococcus aureus. However the activity of silver sulphadiazine in controlling these infections is less than optimum.

Gayle et al, 1978 (Journal of Trauma, 18, 317-323) reported 15 cases of silver sulphadiazine resistant Enterobacter cloacae in one burns unit and 13 of the 15 patients died. Of these deaths 7 were directly caused by, and 2 others significantly related to E. cloacae septicaemia.

An object of the present invention is to enhance the antimicrobial activity of silver compounds suitable for use in the treatment of burn wound infection.

According to the present invention there is provided a pharmaceutical composition comprising in admixture with a pharmaceutically acceptable vehicle or carrier, a therapeutically effective amount of a silver compound suitable for treating burns and a synergistic amount of dibromopropamidine or pharmaceutically acceptable salts thereof.

Preferably the pharmaceutical composition contains silver sulphadiazine and one of dibromopropamidine, dibromopropamidine isethionate B. P. 1980 or dibromopropamidine pamoate. The dibromopropamidine or salts thereof may be present in concentrations of 0.00015 to 0.30 per cent w/w in

- 2 -

pharmaceutical formulations of silver sulphadiazine at 0. 01 to
2. 0 per cent w/w.

Similar concentrations with preparations containing
other silver salts are found to enhance the activity of these
preparations which may infect the burn wound.

The dibromopropamidine salts have been proposed
for use as topical antibacterials (BPC 1980 and Martindale, The
Extra Pharmacopoeia, 1977, 27th Edition, Pharmaceutical
Press, London p. 517) but the synergistic effect exhibited in
combination with silver salts is wholly unexpected. Thus
greater antibacterial activity is obtained against all organisms
tested than is observed with silver salts used alone at equivalent
concentrations. The combination of dibromopropamidine
isethionate 0. 15% with silver sulphadiazine 1. 0% also possesses
activity against the silver resistant Pseudomonas aeruginosa
R 351 against which silver sulphadiazine 1. 0% alone has no
activity. The Minimum Inhibitory Concentration of $[Ag+]$ alone
against this strain of Pseudomonas aeruginosa is 128 $\mu$g ml$^{-1}$.

Examples are given in Table 1 of the in vitro
enhancement of activity when cream formulations were tested
in duplicate by the agar diffusion method against an inoculum
of approximately 5 x 10$^4$ organisms ml$^{-1}$ of test organism. The
test organisms used were Pseudomonas aeruginosa NCTC 6750,
Staphylococcus aureus NCTC 6751, Enterobacter cloacae NCTC
9394 and NCTC 10005.

Typically the compositions of this invention are prepared
as topical antibiotic preparations in for example a water-miscible
cream such as Cetomacrogol cream BPC 1968, or modifications
of such a cream, or into a gel such as can be prepared from a
dispersion of carbomer (Carbopal 940, Honeywell and Stein
Ltd. ) by neutralising with dilute ammonia solution. Gel

TABLE 1

| Enhancement of activity of silver sulphadiazine using combination of silver sulphadiazine and dibromopropamidine isethionate. | | | | |
|---|---|---|---|---|
| CREAM FORMULATION | ORGANISM & ZONE OF INHIBITION (mm). | | | |
| | P. aeruginosa | S. aureus | E. cloacae (10005) | (9394) |
| Silver sulphadiazine 1% | 15.0 | 18.5 | 17.5 | 14.8 |
| Dibromopropamidine isethionate 0.15% | 0 | 24.0 | 26.0 | 19.0 |
| Silver sulphadiazine 1% + Dibromopropamidine 0.15% | 21.0 | 27.0 | 29.0 | Not done |
| Silver sulphadiazine 0.8% + Dibromopropamidine 0.03% | 22.5 | 26.0 | 26.0 | 20.4 |
| Silver sulphadiazine 0.5% + Dibromopropamidine 0.075% | 19.0 | 24.5 | 27.5 | Not done |

- 4 -

formulations show a greater activity than equivalent cream formulations when tested by the agar diffusion method. This activity is shown by larger zones of inhibition of the order of 2-4 mm with the gel formulations. The gel also has the advantage that it can be sterilised by heating in an autoclave at $115^{o}C$ for 30 minutes.

The preparation is applied to the cleansed and debrided burn wound to give a layer approximately 2 to 4 mm thick. If left exposed the cream gel is applied twice daily but when used under an occlusive dressing then it may be applied at daily or longer intervals, according to the condition of the wound, until satisfactory healing has occured or the burn site is ready for grafting.

## Claims

1. A pharmaceutical composition comprising in admixture with a pharmaceutically acceptable vehicle or carrier, a therapeutically effective amount of a silver compound suitable for treating burns and a synergistic amount of dibromopropamidine or pharmaceutically acceptable salts thereof.

2. A composition according to claim 1 which contains silver sulphadiazine and one of dibromopropamidine, dibromopropamidine isethionate B.P. 1980 or dibromopropamidine pamoate.

3. A composition according to claim 2 wherein the dibromopropamidine or salts thereof are present in concentrations of from 0.00015 to 0.30 per cent w/w in pharmaceutical formulations of silver sulphadiazine at 0.01 to 2.0 per cent w/w.

4. A composition according to claim 1 which contains 0.15% dibromopropamidine isethionate and 1% silver sulphadiazine.

5. A composition according to claim 1 wherein the vehicle is an aqueous carbomer dispersion gel.

European Patent
Office

**EUROPEAN SEARCH REPORT**

0125858
Application number

EP   84 30 3055

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| A | US-A-3 761 590   (CHARLES L. FOX) * Column 3, lines 20-33; column 8, lines 24-43; column 10, lines 6-38 * | 1-5 | A 61 K   31/635 // (A 61 K   31/635 A 61 K   31/155) |
| | --- | | |
| A | CHEMICAL ABSTRACTS, vol. 76, no. 13, 27th March 1972, page 63, column 1, no. 68565n, Columbus, Ohio, USA; W.B. HUGO et al.: "Amidines" & INHIBITION DESTRUCT. MICROBIAL CELL 1971, 121-136 * Abstract * | 1-5 | |
| | ---- | | |
| A | GB-A-2 089 659   (UNIVERSITY OF STRATHCLYDE) | 1-5 | |
| | ----- | | **TECHNICAL FIELDS SEARCHED (Int. Cl. ³)** |
| | | | A 61 K |

The present search report has been drawn up for all claims

| Place of search THE HAGUE | Date of completion of the search 13-08-1984 | Examiner BRINKMANN C. |
|---|---|---|